# EUROPEAN PATENT APPLICATION

(11) **EP 1 747 786 A2**
(43) Date of publication of application: **31.01.2007**
(21) Application number: 06015343.4
(22) Date of filing: 24.07.2006
(51) Int. Cl.: A61K 36/00, A61P 17/00

(54) **Natural product in cream with anti-vitiligo therapeutic properties**

(30) Priority: 25.07.2005 VE 149205
(71) Applicant: Perdix Eurogroup S.L., 36680 A Estrada (Pontevedra) (ES)
(72) Inventor: Paleo, Romero Abel Gustavo, Maracaibo Edo. Zulia, 4001 (VE); Rojas, Urdaneta Janeth, Elisabeth, Maracaibo Edo. Zulia, 4001 (VE)

(57) **Abstract**

The aim of this patent is the elaboration of a natural product based on vegetal ingredients with anti-vitiligo therapeutic properties to be employed in the white patches of depigmentation present in the skin with vitiligo and to achieve melanocyte regeneration, which is dysfunctional during the disease. At present, there are evidences indicating a pathogenic association between vitiligo and the increase in epidermal oxidative stress, mechanism underlying the functional alterations in the melanocytes and vitiligo development. It is for this reason that this treatment that we present in this patent application emerges as a natural product with anti-vitiligo therapeutic properties, which contains as active ingredients the watery extract of *Pimienta racemosa*; this active ingredient inhibits neutrophil chemotaxis and superoxide anion production and acts in this way like an antioxidant on the skin with vitiligo, promoting the restoration of the affected area. Moreover, it contains watery extract of melon, which supplies antioxidant enzymes like catalase and superoxide dismutase, these antioxidant enzymes are free radicals scavengers present in melanocytes. Coenzyme Q-10 is other active ingredient, it helps these enzymes to develop their function besides having antioxidant properties, and it plays an important role in the production of cell energy, and therefore it is an important mitochondrial and immunological stimulator. It also contains Pyridoxine, which is necessary for the metabolism of amino acids like tyrosine and phenylalanine which participate in melanin production and the lemon terpene which contains vitamin C or ascorbic acid which also acts as an antioxidant inhibiting free radicals developing. Its formula is given by: 1:1 of watery extract of *Pimienta racemosa;* 1:2 of watery extract of *Cucumis melo*; 100 ml of extract of *Citrus aurantifolia*; 100 mg Coenzyme Q-10 and 100 mg Pyridoxine Chlorhydrate.

## Description

### Technical records:

### Invention field:

The current invention is aimed to the production of a pharmaceutical product based on vegetal ingredients, mainly, built-in cream for topical use whose active ingredients have therapeutic and pharmacologic properties which stimulate and regenerate melanocyte to produce melanin in the white patches of depigmentation present in the skin of patients with vitiligo.

### Invention records:

One of the most studied aspects in vitiligo disease is the cosmetic deformity that this disease causes in the patient and that determines psychological alterations. Several treatments have been proposed, ranging from the use of cosmetics in order to hide hipomelanosis (Goldstein E, Haberman, HF, Menon JA, Pawlowski D. Int J Dermatol 1992:31:229-36) to the use of hydroquinone monobenzyl ether as a local depigmentating in patients with extensive vitiligo (Jimbow K. Therapeutic advances. Dermatol Clin 1998;6:399-407).

Recently, oxidative stress has been considered as the first episode of some diseases like vitiligo. In this context, evidences indicate a pathogenic association between vitiligo and an increase in the epidermal oxidative stress due to an increase in 6-tetrahydrobiopterin (6-BH₄), 7BH₄ (a potent inhibitor of phenylalanine-hydroxylase), and to an increase in the epidermic activity of the monoamine-oxidase A and the subsequent production of H₂O₂ which could generate a quickly oxidation of 6-BH₄ to 6 biopterin, a melanotoxic molecule (Davis MD, Ribeiro P, Tipper J, Kaufman S. Proe Natl Acad Sci USA 1992; 89:10108-10113; Schallreuter KU, Wood JM, Pittelkow MR. Arch Dermatol Res 1998; 288(1):14-8).

Nowadays, it is known that the therapeutic options for vitiligo are limited and long cycles of treatment are often required with tendency to recidive. The current therapeutic modalities include topical corticosteroids, phototherapy (with narrow-band and broadband UVB), photochemistry (psoralen and UVA), and rare time, clinical assays of general immunosuppression (for example, corticosteroids, cyclosporine). Although it is a problem of cosmetic nature, vitiligo can produce a considerable emotional discomfort to affected people, and this justifies the vigorous development of new more efficient treatments. The optimum photochemical therapy with psoralen-UVA (PUVA) needs be administer during months or years, 3 times a week, to reach partial pigmentation in only 30% to 40% of patients (Ortonne J. Psoralcn therapy in vitiligo. Clin Dermatol 1989;7:120-1351). The acute adverse effects (bum, hyperpigmentation, nauseas) and chronic effects (cataracts, skin cancer) have diminished the enthusiasm of the vitiligo treatment with PUVA, and the topical PUVA achieves variable results with high incidence of hyperpigmentation of the normal skin around the affected areas (Westerhof W, Niedweboer-Krobotoba L. Treatment of vitiligo with UV-B radiation vs topical psoralen plus UV-A. Arch Dermatol 1997;133:1525-15252). On the other hand, phototherapies with UVB are still tedious, and the initial repigmentation needs months of diligent application treatment. Moreover, non-affected skin areas suffer an unnecessary exposition to carcinogenic radiation with UVB.

There is currently an anti-vitiligo product in the market which contains enzymes like superoxide dismulase and catalase which remove frcc radicals from the cells, however this treatment has had relevant limitations in the treatment of the lesions throughout the years.

Therefore, it would be desirable to obtain a more complete treatment, this is, a more efficient treatment and one not causing adverse effects which allows eliminate the psychological problems in persons with vitiligo because it promotes melanocyte regeneration and repigmentation of the achromatic skin lesions, improving the disease.

With this aim the present invention focuses on the natural product with anti-vitiligo therapeutic properties that contains natural ingredients with the necessary properties to reach effective anti-vitiligo action because it reduces the free radicals caused by oxidative stress and by other internal and external factors.

### Description of the invention

The current invention is referred to a new treatment for vitiligo, which is a natural product with anti-vitiligo therapeutic activities in cream form for topical use with natural ingredients.

The new product for vitiligo treatment is composed by:
a) Watery extract of *Pimiemta racemosa* 1:1
b) Watery extract of *Cucumis melo 1:2*
c) Watery extract of *Citrus aurantifolia* 100 ml
d) Coenzime Q-10 100 mg
e) Pyridoxine Chlorhydrate 100 mg

It produccs a therapeutic anti-oxidant and regenerator effect of the melanocyte which is dysfunctional in the lesions of skin with vitiligo, recovering the pigmentation in the treated area.

The cream is a natural product to be used in patients with vitiligo, its therapeutic action is based on the active ingredient extracted in watery solution of the sheet of *Pimienta racemosa* which contains flavonoids type eugenol, which reversibly inhibits the nervous activity, as a local anaesthetic. The sensorial nerve fibres and their functions develop an important function in triggering the inflammatory response. The inhibition of the nervous activity and the vascular components of the inflammatory response by Eugenol, as well as the relationship between these elements, can be linked to its possible antiinflammatory effects, which is beneficial for the skin affected status in patients with white patches of depigmentation present in the skin with vitiligo.

Eugenol also inhibits neutrophil chemotaxis and superoxide anion production and acts in this way like an antioxidant. It has been found that Eugenol also acts as a competitive inhibitor of prostaglandin H (PGH) sintetase and avoids the link of arachidonic acid to this enzyme with the subsequent formation of PGH. Prostaglandins (PG) as well as leukotrienes (LT) are important mediators in the inflammatory response.

The effects produced by reactive oxygen species are molecular events related to tisular damage, what obviously happen at the leukodermal lesions of the skin of patients with vitiligo. The studies performed about this flavonoid have shown the antioxidant potential of Eugenol and related compounds (like isoeugenol) to inhibit the lipidic peroxidation induced by oxygen reactive species. Likewise, it inhibits superoxide radical formation in the xanthine-xhantine oxidase system, as well as in the production of hydroxyl radical, preventing the oxidation of Fe²⁺ in the Fenton reaction, which generates this radical, which is one of the most aggressive to tissues because of all the reactions that it triggers. All this chemoprotective property can be due to a scavenger activity of free radicals.

The watery extract of the melon contains anti.-oxidant enzymes like Superoxide Dismutase (SOD) and Catalase. This active ingredient is employed because of the continuous generation of low amounts of reactive oxygen species (ROS), including hydroxyl radical (OH), superoxide anions (O₂⁻) and hydrogen peroxide (H₂O₂) during the aerobic metabolism, as a response to external and internal stimuli. These minimal ROS concentrations can be essential in many processes, like the intracellular response system (which is related to other processes like cellular proliferation and apoptosis), the immunity, and the defence against microorganisms. However, high doses or an inappropriate elimination of ROS originate an oxidative stress, which can produce severe metabolic dysfunctions and damage to biological macromolecules. Therefore, there will exist a relationship among the antioxidant enzymes levels and the three types of messenger molecules (growth factor, prostaglandins and nitric oxide) involved in cellular homeostasis, it means, an equilibrium between the maintenance of the static or constant conditions at the cellular internal medium and the ROS level.

Coenzyme Q-10 is other active ingredient that helps to SOD and catalyse enzymes to develop their function, besides having antioxidant properties, playing a very important role in the production of cellular energy, because of that it is a potent mitochondrial and immunological stimulant, it also contains Pyridoxine, which is necessary for the metabolism of amino acids like tyrosine and phenylalanine which are involved in melanin production.

The watery extract of lemon terpene is extracted from the skin of the lemon, it contains vitamin C or ascorbic acid, which neutralizes the oxygen, scavenges free hydroxyl radicals, scavenges hyperoxide radicals and regenerates the oxidative form of vitamin E. Its molecules act as a protector barrier in the skin.

All these natural active ingredients make that this cream has a therapeutic anti-oxidant and regetlerator effect of the melanocyte which is dysfunctional in the lesions of skin with vitiligo, recovering the pigmentation in the treated area.

This cream was evaluated in a total of 100 patients who were diagnosed with stable vitiligo vulgaris, randomly chosen from three hospitals of Venezuela, its use was compared to placebo, reaching a statistically significant difference between both (P<0.05) with 85% efficiency in the treated sample (Rojas J, Poleo A. *Evaluación de la formulación antioxidante y estimuladora mitorhondrial VitilVenz en piel de pacientes con vitiligo vulgar estahle. Investigación Clinica,* in press).

## Claims

1. Natural product based on vegetal ingredients with artti-vitiligo therapeutic properties to be employed in the white patches of depigmentation present in the skin with vitiligo and to regenerate the melanocyte, which is dysfunctional during the disease. It shows the following composition:
Watery extract of *Pimiemta racemosa* 1:1
Watery extract of *Cucumis melo* 1:2
100 ml. Watery extract of *Citrus aurantifolia*
100 mg Coenzyme Q-10
100 mg Pyridoxine Chlorhydrate

2. This combination confers, in exact amounts, the flavonoid Eugenol, which acts on neutrophil chemotaxis and removing of free radicals and helps undoubtedly to detoxify the melanocyte of the skin with vitiligo and to regenerate its dysfunctional state caused by the oxidative stress obtaining pigmentation with melanin, improving vitiligo.

3. Watery extract of *Cucumis melo* provides the enzymes Catalase and Superoxide Dismutase which catalyze the reaction of destruction of superoxide radicals through their transformation to hydrogen peroxide. Catalase is one of the most efficient known enzymes, as much that it can not be saturated with H₂O₂ in any concentration, catalyzing its conversion to H₂O and O₂ to protect cells from H₂O₂ generated inside them. With donators of H (methanol, ethanol, forrnic acid, phenols,...) shows peroxidase activity.

4. Watery extract of *Citrus aurantifolia* has as active ingredient the lemon terpene that contains Vitamin C or ascorbic acid which shows the property of preventing the development of free radicals, harmful elements that damage cells of the body including skin cells. Its molecules act as protector barrier of the skin.

5. Coenzyme Q-10 is a natural substance, which acts in thousand of biochemical reactions that happen in each cell of the body thousand times each second, among them the melanin production. This coenzyme is diminished in patients with vitiligo. There is a grcat similarity between the antioxidant properties of vitamin E and the ones of the coenzyme Q-10. This one plays a very important role in the production of cell energy, and therefore it is an important mitochondrial and immunological stimulator.

6. Vitamin B6 (Pyridoxine) has an important role in several vital processes. It is necessary for the metabolism of amino acids like tyrosine and phenylalanine which participate in melanin production.

7. It contains 100 g of exeipients: that contains; unibase cream, eucalyptus essential oil, propyleneglycol, glycerine, cetylic alcohol, nipagin and nipazol.
The procedure to use this cream according to the before reinvidications constitutes the natural product in cream with therapeutic properties anti-vitiligo, **characterized by** dysfunctional melanocyte regeneration in the lesions of skin with vitiligo, recovering the pigmentation in the affected area.
